(19) Europäisches Patentamt
European Patent Office
Office européen des brevets

(11) EP 3 919 043 A1

(12) **EUROPEAN PATENT APPLICATION**

(43) Date of publication:
**08.12.2021 Bulletin 2021/49**

(21) Application number: **21180116.2**

(22) Date of filing: **05.01.2016**

(51) Int Cl.:
***A61K 8/26*** *(2006.01)* ***A61K 8/31*** *(2006.01)*
***A61K 8/41*** *(2006.01)* ***A61K 8/58*** *(2006.01)*
***A61K 8/891*** *(2006.01)* ***A61K 8/06*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2015 FR 1550031**

(62) Document number(s) of the earlier application(s) in accordance with Art. 76 EPC:
**16700017.3 / 3 242 651**

(27) Previously filed application:
**05.01.2016 PCT/EP2016/050044**

(71) Applicant: **L'OREAL**
**75008 Paris (FR)**

(72) Inventors:
- **RICARD, Audrey**
**94152 CHEVILLY LARUE (FR)**
- **KONGMANY, Céline**
**94152 CHEVILLY LARUE (FR)**

(74) Representative: **Nony**
**11 rue Saint-Georges**
**75009 Paris (FR)**

Remarks:
This application was filed on 17-06-2021 as a divisional application to the application mentioned under INID code 62.

(54) **COMPOSITION, IN PARTICULAR A COSMETIC MAKEUP AND/OR CARE COMPOSITION, COMPRISING A LIPOPHILIC CLAY, FROM 1% TO 10% BY WEIGHT OF MICA AND AT LEAST ONE NON-CYCLIC SILICONE OIL**

(57) Composition, in particular a cosmetic composition, especially for coating keratin materials, more particularly for making up and/or caring for keratin materials, such as the skin, in the form of a water-in-oil emulsion comprising an aqueous phase, a fatty phase and pigments, characterized in that:
said composition comprises at least one lipophilic clay and at least mica in the form of filler(s), said mica in filler form comprising at least fluorophlogopite,
wherein the composition comprises from 1.2% to 5% by weight of mica relative to the total weight of the composition,
said fatty phase comprises at least one volatile hydrocarbon-based oil and at least one non-cyclic silicone oil,
the volatile hydrocarbon-based oil being present in a content ranging from 5% to 20% by weight relative to the total weight of the composition,
the non-cyclic silicone oil being present in a content ranging from 10% to 25% by weight relative to the total weight of the composition.

EP 3 919 043 A1

## Description

The present invention relates to a composition, in particular a cosmetic composition, especially for coating keratin materials, more particularly for making up and/or caring for keratin materials, such as the skin. Preferably, the invention relates to a composition of fluid foundation type, which has optimized sensory and cosmetic qualities.

Cosmetic compositions, especially foundations, are commonly used to give the skin an aesthetic colour, but also to hide and/or unify skin relief imperfections such as wrinkles and/or fine lines and/or scars; in this regard, coverage is one of the main properties sought.

Consequently, many solid or fluid, anhydrous or non-anhydrous formulations have been developed to date. The present invention is more particularly directed towards fluid compositions in the form of emulsions.

In compositions of emulsion type, coverage is generally obtained by means of pigments. The introduction into said compositions of a large amount of pigments allows a covering and homogeneous makeup result to be obtained, but these properties are obtained at the expense of a natural makeup effect and the cosmetic properties.

Specifically, the properties of the pigments to absorb the oils present in the compositions leads to the production of thicker formulations, which are less easy to spread and which lead to a "mask" effect that is generally unacceptable.

Thus, there is still a need to propose novel formulations for improving the makeup result, especially for obtaining a makeup result that is perceived as being more natural, while at the same time conserving satisfactory coverage.

The technical problem at the heart of the present invention is that of proposing compositions, especially foundations, which have a balance between the technical performance qualities and sensory perceptions generally sought, namely a satisfactory makeup result, a sensation of skin moisturization and a sensation of breathability of the skin. In the specific case of foundations, the term "central foundation" is occasionally used to qualify a composition that combines all of these properties.

The inventors have shown, unexpectedly and advantageously, that the compositions according to the present invention can satisfy this need.

Thus, according to one of its aspects, the present invention is directed towards a composition, in particular a cosmetic composition, especially for coating keratin materials, more particularly for making up and/or caring for keratin materials, such as the skin, in the form of a water-in-oil emulsion comprising an aqueous phase, a fatty phase and pigments, characterized in that:

said composition comprises at least mica in a proportion ranging from 1% to 10% by weight relative to the total weight of the composition and at least one lipophilic clay,
said fatty phase comprises at least one volatile hydrocarbon-based oil and at least one non-cyclic silicone oil,
the volatile hydrocarbon-based oil being present in a content ranging from 5% to 20% by weight relative to the total weight of the composition,
the non-cyclic silicone oil being present in a content ranging from 10% to 25% by weight relative to the total weight of the composition.

The composition according to the invention has good coverage properties: visually, it affords a natural and homogeneous colour effect on application, while at the same time giving the user a sensation of freshness and lightness of feel.

Finally, the composition proves to be easy to apply to the surface of the targeted keratin material. This performance is especially characterized technically by a good play time.

The compositions obtained are stable and homogeneous: no sedimentation or agglomeration of the particles, especially of the pigments, is observed.

As emerges from the examples given below, the inventors have found that the compositions according to the invention lead to the production of a deposit on the skin that is judged to be fine and homogeneous and to a covering and natural makeup result: the colour and relief imperfections are satisfactorily masked.

According to another of its aspects, a subject of the present invention is also a process for coating keratin materials, more particularly for making up and/or caring for keratin materials, such as the skin, characterized in that it comprises the application to the keratin materials of at least one composition in accordance with the invention.

### Cosmetic composition

The composition according to the invention is a water-in-oil emulsion.

An emulsion is generally formed from an oily liquid phase, in this case the continuous phase, and an aqueous liquid phase, in this case the dispersed phase.

It is a dispersion of droplets of aqueous phase in the oily phase. The size of the droplets forming the dispersed aqueous phase of the emulsion is typically about a micrometre (0.1 to 100 μm).

Furthermore, an emulsion requires the presence of a surfactant or of an emulsifier to ensure its stability over time.
According to the present invention, the aqueous phase and the oily phase forming a composition according to the invention are present therein in a weight ratio ranging from 5/50 to 95/50. More preferentially, the aqueous phase and the oily phase are present in a weight ratio ranging from 20/80 to 45/55, preferably 35/65 to 40/60. The ratio between the two phases is adjusted according to the desired cosmetic properties.
Multi-phase formulations may also be developed.
Advantageously, the composition according to the invention has a viscosity, measured at 25°C, at a shear rate of 200 $min^{-1}$ (200 rpm, i.e. a frequency of 50 Hz), ranging from 0.12 to 0.6 Pa.s (1.2 to 6 poises) and especially ranging from 0.25 to 0.49 Pa.s (2.5 to 4.9 poises). Such a viscosity allows easy application of the emulsion and the production of a homogeneous, uniform makeup result with no marks. The viscosity is measured at 25°C, using a Contraves TV viscometer equipped with a No. 2 spindle, the measurement being performed after 10 minutes of rotation of the spindle (after which time stabilization of the viscosity and of the spin speed of the spindle are observed), at a shear rate of 200 $min^{-1}$.

## Mica

The mica used in the composition according to the invention is in the form of pure mica, i.e. in the form of filler(s), in the form of nacre(s), i.e. it is then covered with at least one metal oxide, or in the form of mixtures of filler(s) and nacre(s).
The fillers are colourless or white solid particles of any form, which are in a form that is insoluble and dispersed in the medium of the composition.
These fillers, of mineral or organic, natural or synthetic nature, give the composition containing them softness and give the makeup result a matt effect and uniformity. In addition, these fillers advantageously make it possible to combat various attacking factors such as sebum or sweat.
The mica in filler form preferably used is fluorophlogopite.
The nacres may be chosen from nacreous pigments such as titanium mica covered with an iron oxide, titanium mica covered with bismuth oxychloride, titanium mica covered with chromium oxide or titanium mica covered with an organic dye.
They may also be mica particles, at the surface of which are superposed at least two successive layers of metal oxides and/or of organic dyestuffs.
Examples of nacres that may also be mentioned include natural mica covered with titanium oxide, with iron oxide, with natural pigment or with bismuth oxychloride.
Among the commercially available nacres that may be mentioned are the nacres Timica, Flamenco and Duochrome (on mica base) sold by the company Engelhard, the Timiron nacres sold by the company Merck, the Prestige nacres on mica base sold by the company Eckart and the Sunshine nacres on synthetic mica base sold by the company Sun Chemical.
The nacres may more particularly have a yellow, pink, red, bronze, orange, brown, gold and/or coppery colour or tint.
Advantageously, the nacres in accordance with the invention are micas covered with titanium dioxide or with iron oxide, and also bismuth oxychloride.
The composition according to the invention comprises from 1% to 10%, preferably from 1.2% to 5% and advantageously from 1.5% to 3% by weight of mica relative to the total weight of the composition.

## Lipophilic clays

The composition according to the invention comprises at least one lipophilic clay as gelling agent.
The clays may be natural or synthetic, and they are made lipophilic by treatment with an alkylammonium salt such as a $C_{10}$ to $C_{22}$ ammonium chloride, for example distearyldimethylammonium chloride.
They may be chosen from bentonites, in particular hectorites and montmorillonites, beidellites, saponites, nontronites, sepiolites, biotites, attapulgites, vermiculites and zeolites.
They are preferably chosen from hectorites.
Preferably, the lipophilic clays used are hectorites modified with a $C_{10}$ to $C_{22}$ ammonium chloride, such as hectorite modified with distearyldimethylammonium chloride, for instance the product sold under the name Bentone 38V® by the company Elementis or bentone gel in isododecane sold under the name Bentone Gel ISD V® (87% isododecane/10% disteardimonium hectorite/3% propylene carbonate) by the company Elementis.
The lipophilic clay may especially be present in a content ranging from 0.1% to 15% by weight, in particular from 0.5% to 10% and more particularly from 0.7% to 2% by weight relative to the total weight of the oily phase.

## Oily phase

For the purposes of the invention, an oily phase comprises at least one oil.
The term "*oil"* means any fatty substance that is in liquid form at room temperature and atmospheric pressure.
An oily phase that is suitable for preparing the cosmetic compositions according to the invention may comprise hydrocarbon-based oils, silicone oils, fluoro oils or non-fluoro oils, or mixtures thereof.
The oils may be volatile or non-volatile.
They may be of animal, plant, mineral or synthetic origin.
For the purposes of the present invention, the term "non-volatile oil" means an oil with a vapour pressure of less than 0.13 Pa.
For the purposes of the present invention, the term "silicone oil" means an oil comprising at least one silicon atom, and in particular at least one Si-O group.
The term "*fluoro oil"* means an oil comprising at least one fluorine atom.
The term "*hydrocarbon-based oil"* means an oil mainly containing hydrogen and carbon atoms.
The oils may optionally comprise oxygen, nitrogen, sulfur and/or phosphorus atoms, for example in the form of hydroxyl or acid radicals.
For the purposes of the invention, the term *"volatile oil"* means any oil that is capable of evaporating on contact with the skin in less than one hour, at room temperature and atmospheric pressure. The volatile oil is a volatile cosmetic compound, which is liquid at room temperature, especially having a non-zero vapour pressure, at room temperature and atmospheric pressure, especially having a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

## Volatile hydrocarbon-based oils

The composition according to the invention comprises at least one volatile hydrocarbon-based oil, preferably comprising from 8 to 16 carbon atoms.
Mention may be made of branched $C_8$-$C_{16}$ alkanes, such as $C_8$-$C_{16}$ isoalkanes (also known as isoparaffins), especially isododecane, isodecane, isohexadecane and, for example, the oils sold under the trade names Isopar or Permethyl, branched $C_8$-$C_{16}$ esters, such as isohexyl neopentanoate, and mixtures thereof.
Preferably, the volatile hydrocarbon-based oil is chosen from volatile hydrocarbon-based oils containing from 8 to 16 carbon atoms, and mixtures thereof, in particular from isododecane, isodecane and isohexadecane, and especially isododecane.
Mention may also be made of volatile linear alkanes comprising from 8 to 16 carbon atoms, in particular from 10 to 15 carbon atoms and more particularly from 11 to 13 carbon atoms, for instance n-dodecane ($C_{12}$) and n-tetradecane ($C_{14}$) sold by Sasol under the respective references Parafol 12-97 and Parafol 14-97, and also mixtures thereof, the undecane-tridecane mixture, mixtures of n-undecane ($C_{11}$) and of n-tridecane ($C_{13}$) such as those obtained in Examples 1 and 2 of patent application WO 2008/155 059 from the company Cognis, and mixtures thereof.
Preferably, the volatile hydrocarbon-based oil is present in the composition according to the invention in a content ranging from 5% to 20% by weight or even from 10% to 18% by weight relative to the total weight of the composition.

## Non-cyclic silicone oils

The present invention comprises at least one non-cyclic silicone oil.
The term "silicone oil" means an organopolysiloxane.
The term "*non-cyclic* oil" denotes a linear or branched oil, not comprising any rings. In other words, the oil in accordance with the invention is free of (hetero)aryl radicals and/or of (hetero)cycloalkyl radicals.
The non-cyclic silicone oil may be volatile or non-volatile.
The term "non-volatile oil" means an oil that remains on the keratin fibre at room temperature and atmospheric pressure for at least several hours and that especially has a vapour pressure of less than $10^{-3}$ mmHg (0.13 Pa). A non-volatile oil may also be defined as having an evaporation rate such that, under the conditions defined previously, the amount evaporated after 30 minutes is less than 0.07 mg/cm$^2$.
Preferably, the molecular weight of the non-cyclic silicone oil is between 500 and 100 000 g/mol.
According to the invention, non-cyclic silicone oils having at 25°C a viscosity of between 1 and 20 cSt (between 1 and 20 mm$^2$/s) are particularly suitable for use.
The viscosity measurement method used in the invention to characterize the silicone oils according to the invention may be the "kinematic viscosity at 25°C raw product CID-012-01" or the "Ubbelohde viscosity at 25°C DIN 51562-1 PV04001".

The non-cyclic silicone oils that may be used in the makeup compositions according to the present invention are represented by the general formula (I) below:

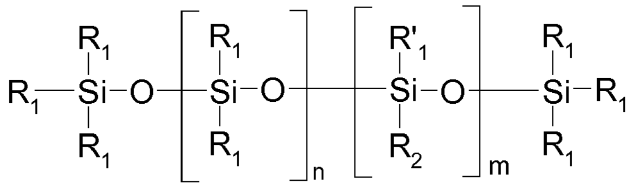

(I)

with:

◦ $\mathbf{R_1}$, which may be identical or different, representing:

- i) a linear or branched ($C_1$-$C_{20}$) alkyl and particularly a $C_1$-$C_6$ alkyl group, such as methyl, ethyl, propyl or butyl; or
- ii) a hydroxyl group;

◦ $\mathbf{R_2}$ representing:

- i) a linear or branched ($C_1$-$C_{20}$)alkyl group optionally interrupted and/or terminated with a heteroatom such as O, S or N; in particular, i) is a linear or branched $C_1$-$C_6$ alkyl group, such as methyl, ethyl, propyl or butyl;
- ii) a group ($C_1$-$C_9$)(poly)haloalkyl, especially perfluoroalkyl, comprising from 1 to 9 halogen atoms, particularly fluorine, such as trifluoromethyl; and
- iii) the polysiloxane group $-O-[Si(R_1)_2-O]_{n'}-Si(R_1)_3$ with $R_1$ as defined previously;

o $\mathbf{R_1}$ representing a radical $R_1$ or $R_2$ as defined previously;
◦ **m** being an integer inclusively between 0 and 150 and preferably between 20 and 100;
◦ **n** and **n',** which may be identical or different, being an integer inclusively between 1 and 300 and preferably between 1 and 100.

According to a preferred embodiment, $R'_1$ represents the radical $R_1$, and more particularly a group ($C_1$-$C_6$)alkyl such as methyl.
According to one particular embodiment, m is 0.
According to another particular embodiment of the invention, $R_1$ is a methyl, and more particularly m is 0 and $R_1$ is a methyl.
According to a particular example of the invention, the non-cyclic silicone oils may be chosen from a fluorosilicone compound.
Fluorosilicone compounds that may especially be mentioned include those sold by the company Shin-Etsu under the names X22-819, X22-820, X22-821 and X22-822 or FL-100.
According to a preferred embodiment, said non-cyclic silicone oil is a dimethicone corresponding to formula (II) below:

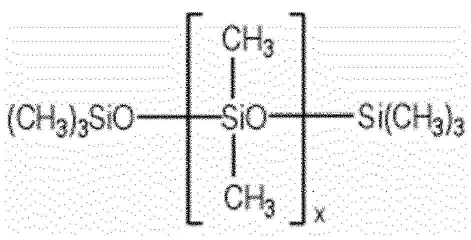

(II)

in this formula (II), x being an integer ranging from 1 to 50, better still from 1 to 20 and more specifically from 1 to 10. The molecular mass of such a compound may be, for example, approximately 770 g/mol. Preferably, x is between 1 and 7, advantageously between 1 and 5.
According to a particular embodiment, the non-cyclic silicone oil of general formula (I) or (II), having at 25°C a

viscosity of between 1 and 20 cSt, is advantageously chosen from the following oils: octamethyltrisiloxane (1 cSt), decamethyltetrasiloxane (1.5 cSt) and dodecamethylpentasiloxane (2 cSt), or alternatively dimethicones with a viscosity equal to 5 cSt, i.e. the oils of INCI name Dimethicone 5 cSt, for instance the products sold by the company Dow Corning under the references 200R Fluid 5 cSt® or Xiameter® PMX-200 Silicone Fluid 5CS®.

The non-cyclic silicone oil is present in the composition according to the invention in a content ranging from 10% to 25% by weight relative to the total weight of the composition.

## Non-volatile oils

The composition according to the invention may comprise a non-volatile oil.

It falls within the competence of a person skilled in the art to select the nature and amount of non-volatile oil(s) that may be introduced into the composition without impairing the properties thereof.

Advantageously, the compositions according to the invention comprise less than 5% by weight, preferably less than 3% by weight, and are preferably free of volatile cyclic silicone oils, for instance octamethylcyclotetrasiloxane and decamethylcyclopentasiloxane.

When they are present, the non-volatile oils may be chosen especially from non-volatile hydrocarbon-based, fluoro and/or silicone oils.

Non-volatile hydrocarbon-based oils that may especially be mentioned include:

- hydrocarbon-based oils of animal origin,
- hydrocarbon-based oils of plant origin, synthetic ethers containing from 10 to 40 carbon atoms, such as dicaprylyl ether,
- synthetic esters, for instance oils of formula $R_1COOR_2$, in which $R_1$ represents a linear or branched fatty acid residue containing from 1 to 40 carbon atoms, and $R_2$ represents a hydrocarbon-based chain that is especially branched, containing from 1 to 40 carbon atoms provided that $R_1 + R_2 \geq 10$. The esters may be chosen especially from fatty acid alcohol esters, for instance cetostearyl octanoate, isopropyl alcohol esters such as isopropyl myristate or isopropyl palmitate, ethyl palmitate, 2-ethylhexyl palmitate, isopropyl stearate, octyl stearate, hydroxylated esters, such as isostearyl lactate or octyl hydroxystearate, alkyl or polyalkyl ricinoleates, hexyl laurate, neopentanoic acid esters, such as isodecyl neopentanoate or isotridecyl neopentanoate, and isononanoic acid esters, such as isononyl isononanoate or isotridecyl isononanoate,
- polyol esters and pentaerythritol esters, such as dipentaerythrityl tetrahydroxystearate/tetraisostearate,
- fatty alcohols that are liquid at room temperature, bearing a branched and/or unsaturated carbon-based chain containing from 12 to 26 carbon atoms, for instance 2-octyldodecanol, isostearyl alcohol and oleyl alcohol,
- higher $C_{12}$-$C_{22}$ fatty acids, such as oleic acid, linoleic acid and linolenic acid, and mixtures thereof,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, and trimethyl-pentaphenyl-trisiloxane, and mixtures thereof; and also mixtures of these various oils.

A composition according to the invention may comprise from 1% to 95% by weight, better still from 1.5% to 40% by weight and preferably from 2% to 35% by weight of oil(s) relative to the total weight of said composition.

## Aqueous phase

The aqueous phase of the composition according to the invention comprises water and optionally a water-soluble solvent.

In the present invention, the term "*water-soluble solvent"* denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms such as ethanol and isopropanol, glycols containing from 2 to 8 carbon atoms such as ethylene glycol, propylene glycol, 1,3-butylene glycol and dipropylene glycol, $C_3$ and $C_4$ ketones and $C_2$-$C_4$ aldehydes.

The aqueous phase (water and optionally the water-miscible solvent) may be present in the composition in a content ranging from 5% to 95%, better still from 30% to 80% by weight and preferably from 40% to 75% by weight relative to the total weight of said composition.

According to another embodiment variant, the aqueous phase of a composition according to the invention may

comprise at least one $C_2$-$C_{32}$ polyol.

For the purposes of the present invention, the term *"polyol"* should be understood as meaning any organic molecule comprising at least two free hydroxyl groups.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are advantageously suitable for formulating a composition according to the present invention are those especially containing from 2 to 32 carbon atoms and preferably 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof.

According to a preferred embodiment of the invention, said polyol is chosen from ethylene glycol, pentaerythritol, trimethylolpropane, propylene glycol, glycerol, polyglycerols, polyethylene glycols and mixtures thereof.

According to a particular mode, the composition of the invention may comprise at least propylene glycol.

According to another particular mode, the composition of the invention may comprise at least glycerol.

## Pigments

The composition according to the invention comprises at least one pigment.

The term *"pigments"* means white or coloured, mineral or organic particles, which are insoluble in an aqueous medium, and which are intended to colour and/or opacify the resulting composition and/or film. These pigments may be white or coloured, and mineral and/or organic.

Preferably, the composition comprises from 0.01% to 25% by weight, especially from 0.1% to 25% by weight, in particular from 1% to 25% by weight and preferably from 5% to 15% by weight of pigments relative to the total weight of said composition.

## Hydrophobic coated pigments

Preferably, the compositions according to the invention comprise at least one pigment coated with at least one lipophilic or hydrophobic compound and especially as detailed below.

This type of pigment is particularly advantageous insofar as it may be considered in large amount together with a large amount of water. What is more, insofar as they are treated with a hydrophobic compound, they show a predominant affinity for the oily gelled phase, which can then convey them.

Needless to say, the compositions according to the invention may in parallel contain uncoated pigments.

These pigments are more particularly detailed below.

According to a particular embodiment, the coated pigments used according to the invention are chosen from mineral pigments.

The term *"mineral pigment"* means any pigment that satisfies the definition in Ullmann's encyclopaedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminium powder or copper powder. The following mineral pigments may also be used: $Ta_2O_5$, $Ti_3O_5$, $Ti_2O_3$, TiO, $ZrO_2$ as a mixture with $TiO_2$, $ZrO_2$, $Nb_2O_5$, $CeO_2$, ZnS.

The size of the pigment that is useful in the context of the present invention is generally between 10 nm and 10 $\mu$m, preferably between 20 nm and 5 $\mu$m and more preferentially between 30 nm and 1 $\mu$m.

In the context of the present invention, the coated mineral pigments are more particularly iron oxide and/or titanium dioxide.

Examples that may be mentioned more particularly include titanium dioxide and iron oxide coated with aluminium stearoyl glutamate, sold, for example, under the reference NAI by the company Miyoshi Kasei.

### Coating of the pigment

The composition according to the invention advantageously comprises at least one pigment coated with at least one lipophilic or hydrophobic compound.

The coating may also comprise at least one additional non-lipophilic compound.

For the purposes of the invention, the "coating" of a pigment according to the invention generally denotes the total or partial surface treatment of the pigment with a surface agent, absorbed, adsorbed or grafted onto said pigment.

The surface-treated pigments may be prepared according to surface treatment techniques of chemical, electronic, mechanochemical or mechanical nature that are well known to those skilled in the art. Commercial products may also be used.

The surface agent may be absorbed, adsorbed or grafted onto the pigments by evaporation of solvent, chemical reaction and creation of a covalent bond.

According to one variant, the surface treatment consists of coating of the pigments.

The coating may represent from 0.1% to 20% by weight and in particular from 0.5% to 5% by weight, relative to the total weight of the coated pigment.

The coating may be performed, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be performed, for example, by chemical reaction of a surface agent with the surface of the solid pigment particles and creation of a covalent bond between the surface agent and the particles. This method is especially described in patent US 4 578 266.

The chemical surface treatment may consist in diluting the surface agent in a volatile solvent, dispersing the pigments in this mixture and then slowly evaporating off the volatile solvent, so that the surface agent is deposited at the surface of the pigments.

Lipophilic or hydrophobic treatment agent

When the pigment comprises a lipophilic or hydrophobic coating, it is preferably present in the fatty phase of the composition according to the invention.

According to a particular embodiment of the invention, pigments may be coated according to the invention with at least one compound chosen from silicone surface agents; fluoro surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

*Silicone surface agent*

According to a particular embodiment, the pigments may be totally or partially surface-treated with a compound of silicone nature.

The silicone surface agents may be chosen from organopolysiloxanes, silane derivatives, silicone-acrylate copolymers, silicone resins, and mixtures thereof.

The term *"organopolysiloxane compound"* means a compound having a structure comprising an alternance of silicon atoms and oxygen atoms and comprising organic radicals linked to silicon atoms.

*i) Non-elastomeric organopolysiloxane*

Non-elastomeric organopolysiloxanes that may especially be mentioned include polydimethylsiloxanes, polymethylhydrogenosiloxanes and polyalkoxydimethylsiloxanes.

The alkoxy group may be represented by the radical R-O- such that R represents methyl, ethyl, propyl, butyl or octyl, 2-phenylethyl, 2-phenylpropyl or 3,3,3-trifluoropropyl radicals, aryl radicals such as phenyl, tolyl or xylyl, or substituted aryl radicals such as phenylethyl.

One method for surface-treating pigments with a polymethylhydrogenosiloxane consists in dispersing the pigments in an organic solvent and then in adding the silicone compound. On heating the mixture, covalent bonds are created between the silicone compound and the surface of the pigment.

According to a preferred embodiment, the silicone surface agent may be a non-elastomeric organopolysiloxane, especially chosen from polydimethylsiloxanes.

*ii) Alkylsilanes and alkoxysilanes*

Silanes bearing alkoxy functionality are especially described by Witucki in "A silane primer, Chemistry and applications of alkoxy silanes, Journal of Coatings Technology, 65, 822, pages 57-60, 1993".

Alkoxysilanes such as the alkyltriethoxysilanes and the alkyltrimethoxysilanes sold under the references Silquest A-137 (OSI Specialities) and Prosil 9202 (PCR) may be used for coating the pigments.

The use of alkylpolysiloxanes bearing a reactive end group such as alkoxy, hydroxyl, halogen, amino or imino is described in patent application JP H07-196946. They are also suitable for treating the pigments.

*iii) Silicone-acrylate polymers*

Grafted silicone-acrylic polymers having a silicone backbone as described in patents US 5 725 882, US 5 209 924, US 4 972 037, US 4 981 903, US 4 981 902 and US 5 468 477 and in patents US 5 219 560 and EP 0 388 582 may be used.

Other silicone-acrylate polymers may be silicone polymers comprising in their structure the unit of formula (I) below:

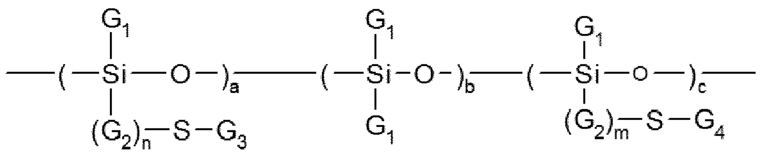

(I)

in which the radicals $G_1$, which may be identical or different, represent hydrogen or a $C_1$-$C_{10}$ alkyl radical or alternatively a phenyl radical; the radicals $G_2$, which may be identical or different, represent a $C_1$-$C_{10}$ alkylene group; $G_3$ represents a polymeric residue resulting from the (homo)polymerization of at least one ethylenically unsaturated anionic monomer; $G_4$ represents a polymeric residue resulting from the (homo)polymerization of at least one ethylenically unsaturated hydrophobic monomer; m and n are equal to 0 or 1; a is an integer ranging from 0 to 50; b is an integer that may be between 10 and 350, c is an integer ranging from 0 to 50; with the proviso that one of the parameters a and c is other than 0.

Preferably, the unit of formula (I) above has at least one, and even more preferentially all, of the following characteristics:

- the radicals $G_1$ denote an alkyl radical, preferably a methyl radical;
- n is non-zero, and the radicals $G_2$ represent a divalent $C_1$-$C_3$ radical, preferably a propylene radical;
- $G_3$ represents a polymeric radical resulting from the (homo)polymerization of at least one monomer of the ethylenically unsaturated carboxylic acid type, preferably acrylic acid and/or methacrylic acid;
- $G_4$ represents a polymeric radical resulting from the (homo)polymerization of at least one monomer of the ($C_1$-$C_{10}$)alkyl (meth)acrylate type, preferably such as isobutyl or methyl (meth)acrylate.

Examples of silicone polymers corresponding to formula (I) are especially polydimethylsiloxanes (PDMS) onto which are grafted, via a connecting chain unit of thiopropylene type, mixed polymer units of the poly(meth)acrylic acid type and of the polymethyl (meth)acrylate type.

Other examples of silicone polymers corresponding to formula (I) are especially polydimethylsiloxanes (PDMS) onto which are grafted, via a connecting chain unit of thiopropylene type, polymer units of the polyisobutyl (meth)acrylate type.

*iv) Silicone resins*

The silicone surface agent may be chosen from silicone resins.

The term *"resin"* means a three-dimensional structure.

The silicone resins may be soluble or swellable in silicone oils. These resins are crosslinked polyorganosiloxane polymers.

The nomenclature of silicone resins is known under the name "MDTQ", the resin being described as a function of the various siloxane monomer units it comprises, each of the letters M, D, T and Q characterizing a type of unit.

The letter M represents the monofunctional unit of formula $(CH_3)_3SiO_{1/2}$, the silicon atom being connected to only one oxygen atom in the polymer comprising this unit.

The letter D means a difunctional unit $(CH_3)_2SiO_{2/2}$ in which the silicon atom is bonded to two oxygen atoms.

The letter T represents a trifunctional unit of formula $(CH_3)SiO_{3/2}$.

In the units M, D and T defined above, at least one of the methyl groups may be substituted with a group R other than a methyl group, such as a hydrocarbon-based radical (especially alkyl) containing from 2 to 10 carbon atoms or a phenyl group, or alternatively a hydroxyl group.

Finally, the letter Q means a tetrafunctional unit $SiO_{4/2}$ in which the silicon atom is bonded to four hydrogen atoms, which are themselves bonded to the rest of the polymer.

Various resins with different properties may be obtained from these different units, the properties of these polymers varying as a function of the type of monomers (or units), of the type and number of substituted radicals, of the length of the polymer chain, of the degree of branching and of the size of the side chains.

Examples of these silicone resins that may be mentioned include:

- siloxysilicates, which may be trimethyl siloxysilicates of formula $[(CH_3)_3XSiXO]_xX(SiO_{4/2})_y$ (MQ units) in which x and y are integers ranging from 50 to 80,
- polysilsesquioxanes of formula $(CH_3SiO_{3/2})_x$ (T units) in which x is greater than 100 and at least one of the methyl radicals of which may be substituted with a group R as defined above,
- polymethylsilsesquioxanes, which are polysilsesquioxanes in which none of the methyl radicals is substituted with another group. Such polymethylsilsesquioxanes are described in document US 5 246 694.

As examples of commercially available polymethylsilsesquioxane resins, mention may be made of those sold:

- by the company Wacker under the reference Resin MK, such as Belsil PMS MK: polymer comprising $CH_3SiO_{3/2}$ repeating units (T units), which may also comprise up to 1% by weight of $(CH_3)_2SiO_{2/2}$ units (D units) and having an average molecular weight of about 10 000, or
- by the company Shin-Etsu under the reference KR220L, which are composed of units T of formula $CH_3SiO_{3/2}$ and have Si-OH (silanol) end groups, under the reference KR242A, which comprise 98% of units T and 2% of dimethyl units D and have Si-OH end groups, or alternatively under the reference KR251 comprising 88% of units T and 12% of dimethyl units D and have Si-OH end groups.

Siloxysilicate resins that may be mentioned include trimethyl siloxysilicate (TMS) resins, optionally in the form of powders. Such resins are sold under the references SRI000, E 1 170-002 or SS 4230, by the company General Electric or under the references TMS 803, Wacker 803 and 804 by the company Wacker Silicone Corporation.

Mention may also be made of trimethylsiloxysilicate resins sold in a solvent such as cyclomethicone, sold under the name KF-7312J by the company Shin-Etsu or DC 749 and DC 593 by the company Dow Corning.

As examples of commercial references of pigments treated with a silicone compound, mention may be made of:

- red iron oxide/dimethicone sold under the reference SA-C 338075-10 by the company Miyoshi Kasei, and
- a pigment obtained by treating DC Red 7 with a silicone compound, sold by the company Coletica under the reference Gransil GCM (which is a mixture of D5 and polysilicone 11).

*Fluoro surface agent*

The pigments may be totally or partially surface-treated with a compound of fluoro nature.

The fluoro surface agents may be chosen from perfluoroalkyl phosphates, perfluoropolyethers, polytetrafluoropolyethylenes (PTFE), perfluoroalkanes, perfluoroalkyl silazanes, polyhexafluoropropylene oxides, and polyorganosiloxanes comprising perfluoroalkyl perfluoropolyether groups.

The term *"perfluoroalkyl radical"* means an alkyl radical in which all of the hydrogen atoms have been replaced with fluorine atoms.

Perfluoropolyethers are especially described in patent application EP 0 486 135, and sold under the trade name Fomblin by the company Montefluos.

Perfluoroalkyl phosphates are described in particular in patent application JP H05-86984. The perfluoroalkyl diethanolamine phosphates sold by Asahi Glass under the reference AsahiGuard AG530 may be used.

Among the linear perfluoroalkanes that may be mentioned are perfluorocycloalkanes, perfluoro(alkylcycloalkanes), perfluoropolycycloalkanes, aromatic perfluoro hydrocarbons (perfluoroarenes) and hydrocarbon-based perfluoro organic compounds comprising at least one heteroatom.

Among the perfluoroalkanes, mention may be made of the linear alkane series such as perfluorooctane, perfluorononane or perfluorodecane.

Among the perfluorocycloalkanes and perfluoro(alkylcycloalkanes), mention may be made of perfluorodecalin sold under the name Flutec PP5 GMP by the company Rhodia, perfluoro(methyldecalin) and perfluoro($C_3$-$C_5$ alkylcyclohexanes) such as perfluoro(butylcyclohexane).

Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives such as tetracosafluorotetradecahydrophenanthrene.

Among the perfluoroarenes, mention may be made of perfluoronaphthalene derivatives, for instance perfluoronaphthalene and perfluoromethyl-1-naphthalene.

As examples of commercial references of pigments treated with a fluoro compound, mention may be made of:

- yellow iron oxide/perfluoroalkyl phosphate sold under the reference PF 5 Yellow 601 by the company Daito Kasei;

- red iron oxide/perfluoroalkyl phosphate sold under the reference PF 5 Red R 516L by the company Daito Kasei;
- black iron oxide/perfluoroalkyl phosphate sold under the reference PF 5 Black BL100 by the company Daito Kasei;
- titanium dioxide/perfluoroalkyl phosphate sold under the reference PF 5 $TiO_2$ CR 50 by the company Daito Kasei;
- yellow iron oxide/perfluoropolymethyl isopropyl ether sold under the reference Iron oxide yellow BF-25-3 by the company Toshiki;
- DC Red 7/perfluoropolymethyl isopropyl ether sold under the reference D&C Red 7 FHC by the company Cardre Inc.; and
- DC Red 6/PTFE sold under the reference T 9506 by the company Warner-Jenkinson.

*Fluorosilicone surface agent*

The pigments may be totally or partially surface-treated with a compound of fluorosilicone nature.
The fluorosilicone compound may be chosen from perfluoroalkyl dimethicones, perfluoroalkyl silanes and perfluoroalkyl trialkoxysilanes.
Perfluoroalkyl silanes that may be mentioned include the products LP-IT and LP-4T sold by Shin-Etsu Silicone.
The perfluoroalkyl dimethicones may be represented by the following formula:

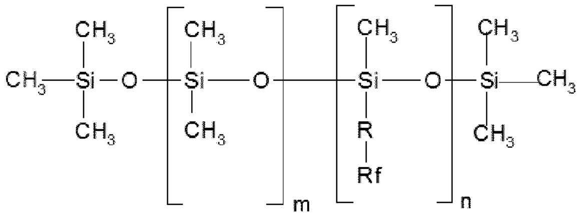

in which:

- R represents a linear or branched divalent alkyl group containing from 1 to 6 carbon atoms, preferably a divalent methyl, ethyl, propyl or butyl group;
- Rf represents a perfluoroalkyl radical containing 1 to 9 carbon atoms and preferably 1 to 4 carbon atoms;
- m is chosen between 0 and 150 and preferably from 20 to 100; and
- n is chosen between 1 and 300 and preferably from 1 to 100.

As examples of commercial references of pigments treated with a fluorosilicone compound, mention may be made of titanium dioxide/fluorosilicone sold under the reference Fluorosil Titanium dioxide 100TA by the company Advanced Dermaceuticals International Inc.

*Other lipophilic surface agents*

The hydrophobic treatment agent may also be chosen from:

i) metal soaps such as aluminium dimyristate and the aluminium salt of hydrogenated tallow glutamate;
Metal soaps that may especially be mentioned include metal soaps of fatty acids containing from 12 to 22 carbon atoms and in particular those containing from 12 to 18 carbon atoms.
The metal of the metal soap may especially be zinc or magnesium.
Metal soaps that may be used include zinc laurate, magnesium stearate, magnesium myristate and zinc stearate, and mixtures thereof;
ii) fatty acids such as lauric acid, myristic acid, stearic acid and palmitic acid;
iii) N-acylamino acids or salts thereof, which may comprise an acyl group containing from 8 to 22 carbon atoms, for instance a 2-ethylhexanoyl, caproyl, lauroyl, myristoyl, palmitoyl, stearoyl or cocoyl group.
The amino acid may be, for example, lysine, glutamic acid or alanine.
The salts of these compounds may be the aluminium, magnesium, calcium, zirconium, zinc, sodium or potassium salts.
Thus, according to a particularly preferred embodiment, an N-acylamino acid derivative may especially be a glutamic acid derivative and/or a salt thereof, and more particularly a stearoyl glutamate, for instance aluminium stearoyl glutamate.
iv) lecithin and derivatives thereof;
v) isopropyl triisostearyl titanate;

As examples of isopropyl titanium triisostearate (ITT)-treated pigments, mention may be made of those sold under the commercial references BWBO-I2 (Iron oxide CI77499 and isopropyl titanium triisostearate), BWYO-I2 (Iron oxide CI77492 and isopropyl titanium triisostearate) and BWRO-I2 (Iron oxide CI77491 and isopropyl titanium triisostearate) by the company Kobo.

vi) isostearyl sebacate;
vii) natural plant or animal waxes or polar synthetic waxes;
viii) fatty esters, in particular jojoba esters;
ix) phospholipids; and
x) mixtures thereof.

The waxes mentioned in the compounds mentioned previously may be those generally used in cosmetics, as defined hereinbelow.

They may especially be hydrocarbon, silicone and/or fluoro waxes, optionally comprising ester or hydroxyl functions. They may also be of natural or synthetic origin.

The term *"polar wax"* means a wax containing chemical compounds comprising at least one polar group. Polar groups are well known to those skilled in the art; they may be, for example, alcohol, ester or carboxylic acid groups. Polyethylene waxes, paraffin waxes, microcrystalline waxes, ozokerite and Fischer-Tropsch waxes are not included among polar waxes.

In particular, the apolar waxes have a mean Hansen solubility parameter $\delta_a$ at 25°C such that $\delta_a > 0$ $(J/cm^3)^{1/2}$ and better still $\delta_a > 1$ $(J/cm^3)^{1/2}$:

$$\delta_a = \sqrt{\delta_p^2 + \delta_h^2}$$

in which $\delta_p$ and $\delta_h$ are, respectively, the polar contributions and contributions of interaction types specific to the Hansen solubility parameters.

The definition of solvents in the three-dimensional solubility space according to Hansen is described in the article by C. M. Hansen: "The three-dimensional solubility parameters", J. Paint Technol. 39, 105 (1967):

- $\delta_h$ characterizes the specific interaction forces (such as hydrogen bonding, acid/base, donor/acceptor, etc.);
- $\delta_p$ characterizes the Debye interaction forces between permanent dipoles and also the Keesom interaction forces between induced dipoles and permanent dipoles.

The parameters $\delta_p$ and $\delta_h$ are expressed in $(J/cm^3)^{1/2}$.

A polar wax is especially formed from molecules comprising, besides carbon and hydrogen atoms in their chemical structure, heteroatoms (such as O, N and P).

Non-limiting illustrations of these polar waxes that may especially be mentioned include natural polar waxes, such as beeswax, lanolin wax, orange wax, lemon wax and Chinese insect waxes, rice bran wax, carnauba wax, candelilla wax, ouricury wax, cork fibre wax, sugarcane wax, Japan wax, sumac wax and montan wax.

According to a particular embodiment, the pigments may be coated with at least one compound chosen from silicone surface agents; fluoro surface agents; N-acylamino acids or salts thereof; isopropyl triisostearyl titanate; natural plant or animal waxes; fatty esters; and mixtures thereof.

According to a particularly preferred embodiment, the pigments may be coated with an N-acylamino acid and/or a salt thereof, in particular with a glutamic acid derivative and/or a salt thereof, or with a fatty ester, in particular with a jojoba ester.

According to a more particularly preferred embodiment, the pigments may be coated with an N-acylamino acid and/or a salt thereof, in particular with a glutamic acid derivative and/or a salt thereof, especially a stearoyl glutamate, for instance aluminium stearoyl glutamate.

Examples of coated pigments according to the invention that may be mentioned more particularly include titanium dioxide and iron oxide coated with aluminium stearoyl glutamate, sold, for example, under the reference NAI by Miyoshi Kasei.

The pigments coated with at least one hydrophobic compound are present in a composition of the invention in a proportion ranging from 5% to 25% and preferably ranging from 10% to 15% by weight relative to the total weight of the composition.

**Pigments not coated with a hydrophobic compound**

As stated previously, a composition may also contain pigments not coated with a lipophilic or hydrophobic compound.

These other pigments may be coated with a hydrophilic compound or not coated.

These pigments may be mineral pigments especially as defined previously.

These pigments may also be organic pigments.

The term *"organic pigment"* means any pigment that satisfies the definition in Ullmann's encyclopaedia in the chapter on organic pigments. The organic pigment may especially be chosen from nitroso, nitro, azo, xanthene, quinoline, anthraquinone, phthalocyanin, metal complex, isoindolinone, isoindoline, quinacridone, perinone, perylene, diketopyrrolopyrrole, thioindigo, dioxazine, triphenylmethane and quinophthalone compounds.

The organic pigment(s) may be chosen, for example, from carmine, carbon black, aniline black, melanin, azo yellow, quinacridone, phthalocyanin blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenolic derivatives as described in patent FR 2 679 771.

These pigments may also be in the form of composite pigments as described in patent EP 1 184 426. These composite pigments may especially be composed of particles comprising a mineral core at least partially covered with an organic pigment and at least one binder for fixing the organic pigments to the core.

The pigment may also be a lake. The term "lake" means insolubilized dyes adsorbed onto insoluble particles, the assembly thus obtained remaining insoluble during use.

The mineral substrates onto which the dyes are adsorbed are, for example, alumina, silica, calcium sodium borosilicate, calcium aluminium borosilicate and aluminium.

Mention may be made, among the organic dyes, of cochineal carmine. Mention may also be made of the products known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090).

An example of a lake that may be mentioned is the product known under the name D&C Red 7 (CI 15 850:1).

*Nature of the hydrophilic coating*

As stated previously, these other pigments may be coated with a hydrophilic compound.

Said hydrophilic compound for surface-treating a pigment in order to optimize its dispersion in the gelled aqueous phase is more particularly chosen from biological polymers, carbohydrates, polysaccharides, polyacrylates and polyethylene glycol derivatives.

As examples of biological polymers, mention may be made of polymers based on monomers of carbohydrate type.

More particularly, mention may be made of biosaccharide gum, chitosans and derivatives thereof, such as butoxy chitosan, carboxymethyl chitosan, carboxybutyl chitosan, chitosan gluconate, chitosan adipate, chitosan glycolate, chitosan lactate, etc., chitins and derivatives thereof, such as carboxymethyl chitin, chitin glycolate; cellulose and derivatives thereof such as cellulose acetate; microcrystalline cellulose; distarch phosphate; sodium hyaluronate; soluble proteoglycans; galacto-arabinans; glycosaminoglycans; glycogen; sclerotium gum; dextran; starch and derivatives thereof; and mixtures thereof.

Examples of carbohydrates that may especially be mentioned include polyhydroxyaldehydes and polyhydroxy ketones of general formula:

$$C_x(H_2O)y$$

in which x and y may range from 1 to 1 000 000.

The carbohydrates may be monosaccharides, disaccharides or polysaccharides.

Examples of carbohydrates that may especially be mentioned include amylodextrins, beta-glucans, cyclodextrins, modified corn starch, glycogen, hyaluronic acid, hydroxypropylcyclodextrin, lactose, maltitol, guanosine, glyceryl starch, *Triticum vulgare* starch, trehalose, sucrose and derivatives thereof, raffinose and sodium chondroitin sulfate.

$C_1$-$C_{20}$ alkylene glycols or $C_1$-$C_{20}$ alkylene glycol ethers, alone or in combination with tri($C_1$-$C_{20}$)alkylsilanes, may also be used as surface treatment agents.

Examples that may be mentioned include pigments surface-treated with PEG alkyl ether alkoxysilane, for instance pigments treated with PEG-8-methyl ether triethoxysilane sold by the company Kobo under the name SW pigments.

Silicones such as dimethicones bearing hydrophilic groups, also known under the name dimethicone copolyols or alkyl dimethicone copolyols, may also be suitable for use in the invention as surface treatment agents. In particular, such dimethicones may comprise, as repeating units, $C_1$-$C_{20}$ alkylene oxides, such as ethylene or propylene oxides.

An example that may be mentioned is the pigment treated with PEG-12-dimethicone, sold by the company Sensient Corporation under the name LCW AQ Pigment.

The amount of pigments coated with at least one hydrophilic compound and/or of uncoated pigments is especially conditioned by the intended use of the cosmetic composition under consideration, and the adjustment of this amount obviously falls within the competence of the composition formulator.

**Particles with a metallic tint**

The composition according to the invention may also comprise at least one particle with a metallic tint.

For the purposes of the present invention, the term *"particles with a metallic tint"* means any compound whose nature, size, structure and surface finish allow it to reflect the incident light, especially in a non-iridescent manner.

The particles with a metallic tint that may be used in the invention are chosen in particular from:

- particles of at least one metal and/or of at least one metal derivative,
- particles comprising a monomaterial or multimaterial, organic or mineral substrate, at least partially coated with at least one layer with a metallic tint comprising at least one metal and/or at least one metal derivative, and
- mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals.

The term *"metal derivatives"* denotes compounds derived from metals, especially oxides, fluorides, chlorides and sulfides.

Illustrations of these particles that may be mentioned include aluminium particles, such as those sold under the names Starbrite 1200 EAC® by the company Siberline and Metalure® by the company Eckart and glass particles coated with a metallic layer, especially those described in documents JP-A-09188830, JP-A-10158450, JP-A-10158541, JP-A-07258460 and JP-A-05017710.

A composition according to the invention may comprise from 0% to 15% by weight of particles with a metallic tint relative to the total weight of said composition.

**Additional fillers**

Advantageously, a composition according to the invention may also comprise one or more additional fillers conventionally used in care and/or makeup compositions and other than micas, pigments and particles with a metallic tint.

These fillers are colourless or white solid particles of any form, which are in a form that is insoluble and dispersed in the medium of the composition.

These fillers, of mineral or organic, natural or synthetic nature, give the composition containing them softness and give the makeup result a matt effect and uniformity. In addition, these fillers advantageously make it possible to combat various attacking factors such as sebum or sweat.

As illustrations of these fillers, mention may be made of talc, silica, kaolin, poly-P-alanine powder and polyethylene powder, powders of tetrafluoroethylene polymers (Teflon®), lauroyllysine, starch, boron nitride, hollow polymer microspheres such as those of polyvinylidene chloride/acrylonitrile, for instance Expancel® (Nobel Industrie), acrylic acid copolymer microspheres, silicone resin microbeads (for example Tospearls® from Toshiba), polyorganosiloxane elastomer particles, precipitated calcium carbonate, magnesium carbonate, magnesium hydrogen carbonate, hydroxyapatite, barium sulfate, aluminium oxides, polyurethane powders, composite fillers, hollow silica microspheres, and glass or ceramic microcapsules. Use may also be made of particles which are in the form of hollow sphere portions, as described in patent applications JP-2003 128 788 and JP-2000 191 789.

In particular, such fillers may be present in a composition according to the invention in a content of between 0.01% and 25% by weight, especially between 0.1% and 20% by weight and in particular between 1% and 10% by weight relative to the total weight of the composition.

## Dispersant

Advantageously, a composition according to the invention may also comprise a dispersant.
Such a dispersant may be a surfactant, an oligomer, a polymer or a mixture of several thereof.
According to a particular embodiment, a dispersant in accordance with the invention is a surfactant.

## Active agent

For a particular care application, a composition according to the invention may comprise at least one moisturizer, also known as a humectant.
Preferably, the moisturizer is glycerol.
The moisturizer(s) may be present in the composition in a content ranging from 0.1% to 15% by weight, especially from 0.5% to 10% by weight or even from 1% to 8% by weight relative to the total weight of said composition.
As other active agents that may be used in the composition of the invention, examples that may be mentioned include vitamins and sunscreens, and mixtures thereof.
Preferably, a composition according to the invention comprises at least one active agent.
It is a matter of routine operations for a person skilled in the art to adjust the nature and the amount of the additives present in the compositions in accordance with the invention such that the desired cosmetic properties thereof are not thereby affected.
According to another embodiment, a composition of the invention may advantageously be in the form of a makeup base composition.
According to another embodiment, a composition of the invention may advantageously be in the form of a foundation.
Such compositions are especially prepared according to the general knowledge of a person skilled in the art.
Throughout the description, including the claims, the term *"comprising a"* should be understood as being synonymous with *"comprising at least one"*, unless otherwise specified.
The terms *"between... and..."* and *"ranging from... to..."* should be understood as being inclusive of the limits, unless otherwise specified.
The invention is illustrated in greater detail by the examples presented below. Unless otherwise mentioned, the amounts indicated are expressed as mass percentages.

## EXAMPLES

In the tables that follow, the amount of each compound is given as weight%/total weight of the composition.

## Preparation of the compositions

**Compositions:**

| | Compounds | Example 1 In accordance | Example 2 In accordance | Example 3 Comparative |
|---|---|---|---|---|
| Phase A1 | Iron oxide (and) disodium stearoyl glutamate (and) aluminium hydroxide, yellow (Miyoshi Kasei) | 2 | 1.88 | 1.88 |
| | Iron oxide (and) disodium stearoyl glutamate (and) aluminium hydroxide, red (Miyoshi Kasei) | 0.75 | 0.70 | 0.70 |
| | Iron oxide (and) disodium stearoyl glutamate (and) aluminium hydroxide, black (Miyoshi Kasei) | 0.5 | 0.47 | 0.47 |
| | Iron oxide (and) disodium stearoyl glutamate (and) aluminium hydroxide, white (Miyoshi Kasei) | 9.55 | 8.95 | 8.95 |

(continued)

| | Compounds | Example 1 In accordance | Example 2 In accordance | Example 3 Comparative |
|---|---|---|---|---|
| LIPOPHILIC PHASE A2 | Disteardimonium hectorite | 1.13 | 1.13 | 1.14 |
| | Synthetic fluorophlogopite (Synafil S1050 from Eckart) | 1.33 | 1.33 | - |
| | Phenoxyethanol | 1 | 1 | 1 |
| | Mica-titanium oxide (nacre) (Flamenco Summit Gold Y30D from BASF Personal Care Ingredients) | 1.22 | - | - |
| | Cetyl PEG/PPG-10/1 dimethicone | 0.8 | 0.84 | 0.83 |
| | PEG-10 Dimethicone | 5.12 | 5.12 | 5.2 |
| | Isododecane | 17.31 | 17.51 | 17.51 |
| | Polyglyceryl+isostearate | 0.61 | 0.61 | 0.62 |
| | Dodecamethylpentasiloxane (Dimethicone 2 cSt) | 22.61 | 23.4 | 23.58 |
| HYDROPHILIC PHASE B | Butylene glycol | 3.78 | 3.78 | 3.84 |
| | Deionized water | qs 100 | qs 100 | qs 100 |
| | Glycerol | 7.17 | 7.28 | 7.28 |
| | Evaluation | +++ | + | - |

**Preparation:**

To prepare phase A1, the pigments are ground in half of the dodecamethylpentasiloxane, and the iron oxides are ground three times in a three-roll mill.

The components of phase A2 are weighed out and mixed together with stirring so as to obtain a homogeneous phase.

Phase A1 is introduced with stirring into phase A2 to obtain phase A.

The aqueous phase B is emulsified in phase A with stirring for 15 minutes at 2500 rpm.

**Results**

A panel of five people tested, comparatively, these foundations on half a face. 0.10 g of a composition was applied to half a face, and 0.10 g of another composition was applied to the other half of the face.

The cosmetic qualities of the formulations on application, i.e. the visual appearance and the perception to the touch, and also the makeup result, were evaluated.

Compositions 1 and 2 were judged to be satisfactory: they gave a deposit judged to be finer and more homogeneous than that obtained using comparative composition 3.

In addition, composition 1 produces a natural makeup that covers without marking the dry areas or reliefs of the face.

Composition 1 corresponds to the composition preferred by the testers.

**Claims**

1. Composition, in particular a cosmetic composition, especially for coating keratin materials, more particularly for making up and/or caring for keratin materials, such as the skin, in the form of a water-in-oil emulsion comprising an aqueous phase, a fatty phase and pigments, **characterized in that**:

    said composition comprises at least one lipophilic clay and at least mica in the form of filler(s), said mica in filler form comprising at least fluorophlogopite,

wherein the composition comprises from 1.2% to 5% by weight of mica relative to the total weight of the composition,
said fatty phase comprises at least one volatile hydrocarbon-based oil and at least one non-cyclic silicone oil,
the volatile hydrocarbon-based oil being present in a content ranging from 5% to 20% by weight relative to the total weight of the composition,
the non-cyclic silicone oil being present in a content ranging from 10% to 25% by weight relative to the total weight of the composition.

**2.** Composition according to the preceding claim, in which the non-cyclic silicone oil has at 25°C a viscosity of between 1 and 20 cSt.

**3.** Composition according to the preceding claim, in which the non-cyclic silicone oil is a dimethicone corresponding to formula (II) below:

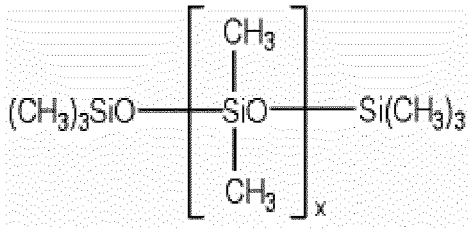
(II)

x being an integer ranging from 1 to 50.

**4.** Composition according to the preceding claim, in which the non-cyclic silicone oil is chosen from octamethyltrisiloxane, decamethyltetrasiloxane and dodecamethylpentasiloxane or dimethicones with a viscosity equal to 5 cSt.

**5.** Composition according to one of the preceding claims, in which the volatile hydrocarbon-based oil comprises from 8 to 16 carbon atoms.

**6.** Composition according to the preceding claim, in which the volatile hydrocarbon-based oil is chosen from branched $C_8$-$C_{16}$ alkanes and branched $C_8$-$C_{16}$ esters and mixtures thereof.

**7.** Composition according to the preceding claim, in which the volatile hydrocarbon-based oil is chosen from volatile linear alkanes comprising from 8 to 16 carbon atoms and also mixtures thereof.

**8.** Composition according to one of the preceding claims, in which the volatile hydrocarbon-based oil is present in a content ranging from 10% to 18% by weight relative to the total weight of the composition.

**9.** Composition according to one of the preceding claims, in which the lipophilic clay is chosen from hectorites modified with a $C_{10}$ to $C_{22}$ ammonium chloride.

**10.** Composition according to one of the preceding claims, in which the lipophilic clay is present in a content ranging from 0.1% to 15% by weight, in particular from 0.5% to 10% and more particularly from 0.7% to 2% by weight relative to the total weight of the composition.

**11.** Composition according to any one of the preceding claims, containing mica in a proportion ranging from 1.5% to 3% by weight relative to the total weight of the composition.

**12.** Composition according to any one of the preceding claims, comprising at least one pigment coated with at least one lipophilic or hydrophobic compound.

**13.** Composition according to any one of the preceding claims, which is in the form of a foundation.

**14.** Process for coating keratin materials **characterized in that** it comprises the application to the keratin materials of at least one composition according to any one of the preceding claims.

Europäisches Patentamt
European Patent Office
Office européen des brevets

# EUROPEAN SEARCH REPORT

Application Number
EP 21 18 0116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2004/103322 A1 (OREAL [FR]; GARDEL NADIA [FR]; BARROIS VERONIQUE [FR]; CHAMPENOIS SAND) 2 December 2004 (2004-12-02) * example 16 * * page 2, lines 25-38 * * page 4, lines 11-18 * ----- | 1-14 | INV. A61K8/26 A61K8/31 A61K8/41 A61K8/58 A61K8/891 A61K8/06 |
| Y | FR 2 891 144 A1 (OREAL [FR]) 30 March 2007 (2007-03-30) * claims 1,24 * * page 23, lines 23-31 * * example 8 * ----- | 1-14 | |
| Y | EP 1 479 366 A1 (OREAL [FR]) 24 November 2004 (2004-11-24) * claim 1 * * page 8, paragraph 78 * * example 1 * ----- | 1-14 | |
| Y | US 2013/302385 A1 (MUENZ SARA [US] ET AL) 14 November 2013 (2013-11-14) * page 8, paragraph 97; example 1 * ----- | 1-14 | TECHNICAL FIELDS SEARCHED (IPC) |
| A | WO 99/66883 A2 (COLOR ACCESS INC [US]) 29 December 1999 (1999-12-29) * the whole document * ----- | 1-14 | A61K A61Q |
| A | US 2005/112072 A1 (WANG JAMES J [US] ET AL) 26 May 2005 (2005-05-26) * the whole document * ----- | 1-14 | |
| A | EP 2 520 336 A2 (BCM COSMETIC GMBH [DE]) 7 November 2012 (2012-11-07) * paragraph [0024] * ----- | 1-14 | |
| | -/-- | | |

The present search report has been drawn up for all claims

1

| Place of search | Date of completion of the search | Examiner |
|---|---|---|
| Munich | 25 October 2021 | Tullberg, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 18 0116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 481 394 A1 (SHISEIDO CO LTD [JP]) 1 August 2012 (2012-08-01) * paragraph [0008] * ----- | 1-14 | |
| A | WO 2013/106999 A1 (OREAL [FR]; LEMOINE CYRIL [CN]; CHU JUAN [CN]) 25 July 2013 (2013-07-25) * the whole document * ----- | 1-14 | |
| A | JP 2001 335432 A (SHISEIDO CO LTD) 4 December 2001 (2001-12-04) * the whole document * ----- | 1-14 | |

TECHNICAL FIELDS SEARCHED (IPC)

1

The present search report has been drawn up for all claims

| Place of search | Date of completion of the search | Examiner |
|---|---|---|
| Munich | 25 October 2021 | Tullberg, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 18 0116

This annex lists the patent family members relating to the patent documents cited in the above-mentioned European search report. The members are as contained in the European Patent Office EDP file on The European Patent Office is in no way liable for these particulars which are merely given for the purpose of information.

25-10-2021

| Patent document cited in search report | Publication date | Patent family member(s) | Publication date |
|---|---|---|---|
| WO 2004103322 A1 | 02-12-2004 | AT 367147 T | 15-08-2007 |
| | | AT 532501 T | 15-11-2011 |
| | | CN 1835732 A | 20-09-2006 |
| | | DE 602004007577 T2 | 03-04-2008 |
| | | EP 1479366 A1 | 24-11-2004 |
| | | EP 1631249 A1 | 08-03-2006 |
| | | ES 2290642 T3 | 16-02-2008 |
| | | ES 2376236 T3 | 12-03-2012 |
| | | FR 2855043 A1 | 26-11-2004 |
| | | JP 4177785 B2 | 05-11-2008 |
| | | JP 2004346075 A | 09-12-2004 |
| | | JP 2006528956 A | 28-12-2006 |
| | | US 2005002890 A1 | 06-01-2005 |
| | | US 2006024251 A1 | 02-02-2006 |
| | | WO 2004103322 A1 | 02-12-2004 |
| FR 2891144 A1 | 30-03-2007 | NONE | |
| EP 1479366 A1 | 24-11-2004 | AT 367147 T | 15-08-2007 |
| | | AT 532501 T | 15-11-2011 |
| | | CN 1835732 A | 20-09-2006 |
| | | DE 602004007577 T2 | 03-04-2008 |
| | | EP 1479366 A1 | 24-11-2004 |
| | | EP 1631249 A1 | 08-03-2006 |
| | | ES 2290642 T3 | 16-02-2008 |
| | | ES 2376236 T3 | 12-03-2012 |
| | | FR 2855043 A1 | 26-11-2004 |
| | | JP 4177785 B2 | 05-11-2008 |
| | | JP 2004346075 A | 09-12-2004 |
| | | JP 2006528956 A | 28-12-2006 |
| | | US 2005002890 A1 | 06-01-2005 |
| | | US 2006024251 A1 | 02-02-2006 |
| | | WO 2004103322 A1 | 02-12-2004 |
| US 2013302385 A1 | 14-11-2013 | NONE | |
| WO 9966883 A2 | 29-12-1999 | AT 321523 T | 15-04-2006 |
| | | AU 764374 B2 | 14-08-2003 |
| | | CA 2300179 A1 | 29-12-1999 |
| | | DE 69930632 T2 | 11-01-2007 |
| | | EP 1047371 A1 | 02-11-2000 |
| | | ES 2262324 T3 | 16-11-2006 |
| | | HK 1031695 A1 | 22-06-2001 |
| | | JP 4213347 B2 | 21-01-2009 |
| | | JP 2002518420 A | 25-06-2002 |
| | | KR 20010023210 A | 26-03-2001 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 21 18 0116

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2021

| Patent document cited in search report | Publication date | Patent family member(s) | Publication date |
|---|---|---|---|
| | | US 6117435 A | 12-09-2000 |
| | | WO 9966883 A2 | 29-12-1999 |
| US 2005112072 A1 | 26-05-2005 | NONE | |
| EP 2520336 A2 | 07-11-2012 | DE 102011050065 A1 | 08-11-2012 |
| | | EP 2520336 A2 | 07-11-2012 |
| EP 2481394 A1 | 01-08-2012 | AU 2010299139 A1 | 03-05-2012 |
| | | BR 112012006666 A2 | 03-05-2016 |
| | | CN 102573783 A | 11-07-2012 |
| | | EP 2481394 A1 | 01-08-2012 |
| | | ES 2543469 T3 | 19-08-2015 |
| | | HK 1168768 A1 | 11-01-2013 |
| | | JP 5041551 B2 | 03-10-2012 |
| | | JP 2011068598 A | 07-04-2011 |
| | | KR 20120078698 A | 10-07-2012 |
| | | RU 2012113125 A | 27-10-2013 |
| | | TW 201110991 A | 01-04-2011 |
| | | US 2012220670 A1 | 30-08-2012 |
| | | WO 2011037123 A1 | 31-03-2011 |
| WO 2013106999 A1 | 25-07-2013 | KR 20140113732 A | 24-09-2014 |
| | | WO 2013106999 A1 | 25-07-2013 |
| | | WO 2013107352 A1 | 25-07-2013 |
| JP 2001335432 A | 04-12-2001 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008155059 A
- US 4578266 A
- JP H07196946 B
- US 5725882 A
- US 5209924 A
- US 4972037 A
- US 4981903 A
- US 4981902 A
- US 5468477 A
- US 5219560 A
- EP 0388582 A
- US 5246694 A
- EP 0486135 A
- JP H0586984 B
- FR 2679771
- EP 1184426 A
- JP 09188830 A
- JP 10158450 A
- JP 10158541 A
- JP 07258460 A
- JP 05017710 A
- JP 2003128788 B
- JP 2000191789 B


### Non-patent literature cited in the description


- **WITUCKI.** A silane primer, Chemistry and applications of alkoxy silanes. *Journal of Coatings Technology,* 1993, vol. 65 (822), 57-60
- **C. M. HANSEN.** The three-dimensional solubility parameters. *J. Paint Technol.,* 1967, vol. 39, 105